# EUROPÄISCHE PATENTANMELDUNG

(11) **EP 2 489 315 A1**
(43) Veröffentlichungstag der Anmeldung: **22.08.2012**
(21) Anmeldenummer: 12000834.7
(22) Anmeldetag: 09.02.2012
(51) Int. Cl.: A61B 17/29

(54) **Medizinisches Instrument**

(30) Priorität: 15.02.2011 DE 102011011244
(71) Anmelder: Karl Storz GmbH & Co. KG, 78532 Tuttlingen (DE)
(72) Erfinder: Blocher, Martin, 78333 Stockach-Espasingen (DE); Bacher, Uwe, 78352 Tuttlingen (DE)
(74) Vertreter: Hofmeister, Frank

(57) **Zusammenfassung**

Die Erfindung betrifft ein medizinisches Instrument (1) mit einem hohlen Schaft (2) und einer am proximalen Ende des Schaftes (2) angeordneten Handhabe (5), wobei der Schaft (2) über einen Antrieb (7) um seine Längsachse (6) verdrehbar ist, wobei der Antrieb (7) als sowohl um die Längsachse (6) des Schaftes (2) verdrehbares als auch in Längsrichtung des Schaftes (2) verlagerbares Stellrad (8) ausgebildet ist. Um ein medizinisches Instrument (1) zu schaffen, dessen Antrieb (7) eine einfache und sichere Handhabung der Schaftverdrehung ermöglicht, wird erfindungsgemäß vorgeschlagen, dass der Antrieb (7) unabhängig von einer anderweitigen Betätigung der Handhabe (5) in jeder Arbeitsposition im Einhandbetrieb betätigbar ist.

## Beschreibung

Die Erfindung betrifft ein medizinisches Instrument mit einem hohlen Schaft und einer am proximalen Ende des Schaftes angeordneten Handhabe, wobei der Schaft über einen Antrieb um seine Längsachse verdrehbar ist und, wobei der Antrieb als sowohl um die Längsachse des Schaftes verdrehbares als auch in Längsrichtung des Schaftes verlagerbares Stellrad ausgebildet ist.

Bei medizinischen Instrumenten mit einem gebogenen Schaft ist es vorteilhaft, dass der Schaft über einen Antrieb um die Längsachse des Schaftes verdrehbar ist, um ohne Verdrehung der Haltehand eine stets optimale Ausrichtung des medizinischen Instruments zum Operationsgebiet zu gewährleisten.

Ein gattungsgemäßes medizinisches Instrument ist beispielsweise aus der DE 102 14 810 A1 bekannt. Dieses als Zange ausgebildete Instrument weist einen Schaft 4 auf, an dessen proximalem Ende eine Handhabe und an dessen distalem Ende ein Zangenmaul angeordnet ist. Um das Zangenmaul um die Längsachse verdrehen zu können, ist am distalen Ende der Handhabe ein Drehrad angeordnet. Durch Drehen des Drehrades lassen sich der Schaft und das Zangenmaul um die Längsachse verdrehen. Weiterhin weist diese Zange eine Verdrehsicherung zwischen dem Drehrad und dem Gehäuse der Handhabe auf, die als beidseitige ringförmige Stirnverzahnung ausgebildet ist.

Diese Verdrehsicherung wird nur wirksam, wenn der verschwenkbare Griffteil der Handhabe zum Betätigen des Zangenmauls betätigt wird. Hierdurch soll verhindert werden, dass das Zangenmaul in der geschlossenen Greifstellung um die Längsachse verdreht werden kann. In der Praxis ist es jedoch teilweise erforderlich, auch in der geschlossenen Position der Maulteile die Lage der Maulteile zu ändern, was aufgrund der Verdrehsicherung bei diesem bekannten Instrument wieder nur durch ein Verdrehen der Haltehand erfolgen kann.

Ein weiteres medizinisches Instrument ist beispielsweise aus der DE 37 11 377 C2 bekannt. Bei diesem bekannten Instrument ist der Antrieb als mit samt dem Schaft um die Längsachse des Schaftes verdrehbares Stellrad ausgebildet. Um einzelne Verdrehschritte einstellen zu können, ist eine federbelastete Kugel vorgesehen, die in entsprechende Vertiefungen im Stellrad eingreift. Diese bekannte Konstruktion ermöglicht zwar ein einfaches Verdrehen des Schaftes sogar im Einhandbetrieb, jedoch bietet die federbelastete Rastkugel keine ausreichende Sicherheit gegen ein versehentliches Verdrehen des Schaftes.

Davon ausgehend liegt der Erfindung die **Aufgabe** zugrunde, ein medizinisches Instrument der eingangs genannten Art zu schaffen, dessen Antrieb eine einfache und sichere Handhabung der Schaftverdrehung ermöglicht.

Die **Lösung** dieser Aufgabenstellung ist durch die Gesamtheit der Merkmale des Anspruchs 1 gekennzeichnet, insbesondere ist der Antrieb unabhängig von einer anderweitigen Betätigung der Handhabe in jeder Arbeitsposition im Einhandbetrieb betätigbar.

Durch die erfindungsgemäße Ausgestaltung des Antriebs als sowohl verdrehbares als auch axial verschiebbares Stellrad, das unabhängig von einer anderweitigen Betätigung der Handhabe in jeder Arbeitsposition im Einhandbetrieb betätigbar ist, ist es erstmalig möglich, einerseits eine zuverlässige Arretierung des Stellrads in der jeweiligen Verdrehstellung des Schaftes zu gewährleisten und andererseits ein Verdrehen des Schaftes auch dann vornehmen zu können, wenn die Handhabe bereits betätigt wurde.

Mit einer ersten Ausführungsform der Erfindung wird vorgeschlagen, dass der Antrieb im Bereich des distalen Endes der Handhabe angeordnet ist.

Gemäß einer alternativen zweiten erfindungsgemäßen Ausführungsform wird vorgeschlagen, dass der Antrieb im Bereich des proximalen Endes des Schaftes angeordnet ist. In beiden Fällen ist es erforderlich, dass der Antrieb, beispielsweise durch eine kraftschlüssige oder formschlüssige Verbindung, so mit dem Schaft zusammenwirkt, dass durch das Betätigen des Antriebs, beispielsweise das Verdrehen des Stellrads, der Schaft um seine Längsachse verdreht werden kann.

Gemäß einer praktischen Ausführungsform der Erfindung wird vorgeschlagen, dass das Stellrad gegen die Kraft mindestens eines Federelements in Längsrichtung des Schaftes verlagerbar ist, um eine zusätzliche Sicherheit gegen ein versehentliches Verdrehen des Stellrads und somit auch des Schaftes zu schaffen. Vorzugsweise ist das Stellrad über das mindestens eine Federelement in Richtung auf die den Schaft arretierende Stellung vorgespannt, so dass das Stellrad automatisch in die arretierende Stellung zurückkehrt, wenn keine Kraft in axialer Richtung mehr auf das Stellrad ausgeübt wird.

Das Arretieren des Stellrads in der jeweiligen Verdrehposition des Schaftes erfolgt erfindungsgemäß über am Stellrad und an der Handhabe ausgebildete, miteinander korrespondierende Rastelemente, über die der Schaft in vorgegebenen Raststufen um die Längsachse des Schaftes verdrehbar an der Handhabe festlegbar ist.

Mit einer bevorzugten Ausführungsform der Erfindung wird vorgeschlagen, dass die Rastelemente koaxial um die Längsachse des Schaftes angeordnet sind, wobei die Rastelemente vorzugsweise als am Stellrad angeordnete Raststifte oder Rastnasen und an der Handhabe ausgebildete Rastnuten ausgebildet sind.

Die Rastnuten sind gemäß einer praktischen Ausgestaltungsform der Erfindung in einer am distalen Ende der Handhabe angeordneten Rastscheibe ausgebildet.

Schließlich wird mit der Erfindung vorgeschlagen, dass der Schaft zumindest abschnittweise gegenüber der Längsachse des Schaftes abgewinkelt ausgebildet ist.

Zur Anordnung des für die Verdrehung des Schaftes benötigten Antriebs bieten sich erfindungsgemäß zwei Ausführungsformen an, nämlich die Anordnung des Antriebs im Bereich des distalen Endes der Handhabe oder alternativ die Anordnung des Antriebs im Bereich des proximalen Endes des Schaftes.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine Seitenansicht eines erfindungsgemäßen medizinischen Instruments;
- Fig. 2: einen Schnitt entlang der Linie II-II gemäß Fig. 1;
- Fig. 3: eine vergrößerte Darstellung des Details III gemäß Fig. 2, den Antrieb in der arretierten Position darstellend;
- Fig. 4: eine Darstellung gemäß Fig. 3, jedoch den Antrieb in der verdrehbaren Position darstellend;
- Fig. 5: eine vergrößerte Darstellung einer ersten Ausführungsform eines erfindungsgemäßen Stellrads und
- Fig. 6: eine vergrößerte Darstellung einer zweiten Ausführungsform eines erfindungsgemäßen Stellrads.

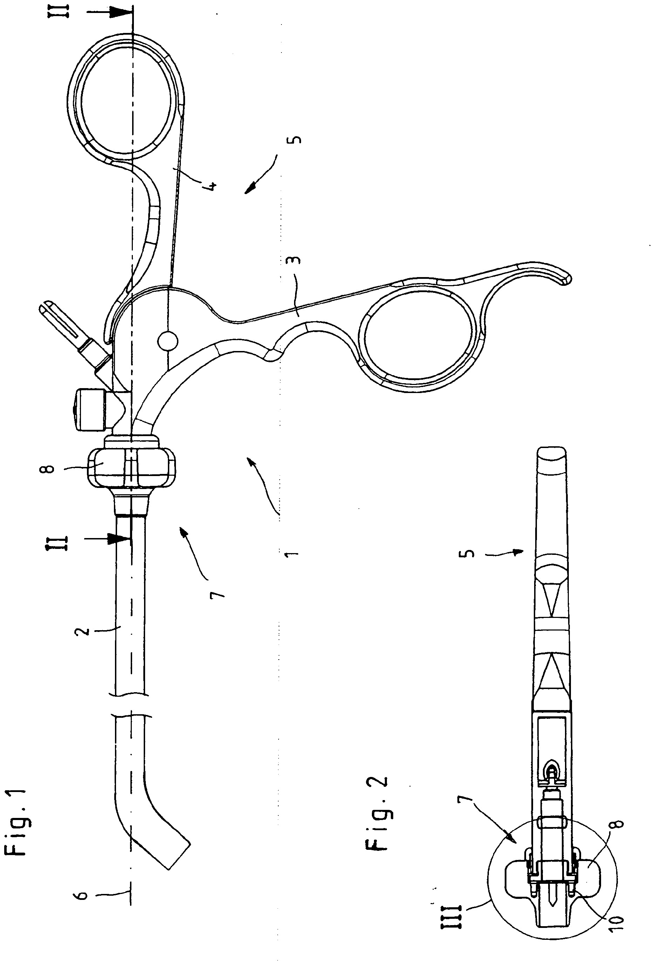

Das in Abbildung Fig. 1 dargestellte medizinische Instrument 1 für endoskopische Zwecke besteht im Wesentlichen aus einem hohlen Schaft 2, an dessen proximalem Ende eine aus zwei Griffteilen 3 und 4 bestehende Handhabe 5 angeordnet ist. Wie weiterhin aus Fig. 1 ersichtlich, ist der Schaft 2 im Bereich seines distalen Endes gegenüber der Längsachse 6 des Schaftes 2 abgewinkelt ausgebildet.

Um ohne ein Verdrehen der Haltehand eine stets optimale Ausrichtung des gebogenen Schaftes 2 des medizinischen Instruments 1 zum Operationsgebiet zu gewährleisten, ist der Schaft 2 über einen Antrieb 7 um seine Längsachse 6 verdrehbar an der Handhabe 5 gelagert. Der Antrieb 7 ist dabei so im Bereich des distalen Endes der Handhabe 5 angeordnet, dass der Benutzer des medizinischen Instruments 1 den Antrieb 7 im Einhandbetrieb mit der die Handhabe 5 ergreifenden Haltehand bedienen kann.

Das Verdrehen des Schaftes 2 des medizinischen Instruments 1 um seine Längsachse 6, kann auch bei einem geraden Schaft 2, also einem Schaft 2 ohne distale Abwinklung sinnvoll sein, wenn beispielsweise das distale Ende des Schaftes 2 nicht gerade, sondern, beispielsweise unter 45° gegenüber der Längsachse 6 abgeschrägt oder löffelförmig, ausgebildet ist. Auch bei einer solchen nicht-geraden Ausbildung des distalen Endes des ansonsten geraden Schaftes 2 kann es in der Praxis erforderlich sein, den Schaft 2 bzw. dessen distales Ende gegenüber der Handhabe 5 auszurichten.

Bei der dargestellten Ausführungsform ist der Antrieb 7 als am distalen Ende der Handhabe 5 angeordnetes Stellrad 8 ausgebildet. Alternativ zu dieser Ausgestaltungsform ist es selbstverständlich auch möglich, den Antrieb 7 am proximalen Ende des Schaftes 2 im Übergang zur Handhabe 5 anzuordnen. In allen Fällen ist es erforderlich, dass der Antrieb 7, beispielsweise durch eine kraftschlüssige oder formschlüssige Verbindung, so mit dem Schaft 2 zusammenwirkt, dass durch das Betätigen des Antriebs 7, beispielsweise das Verdrehen des Stellrads 8, der Schaft 2 um seine Längsachse 6 verdreht werden kann.

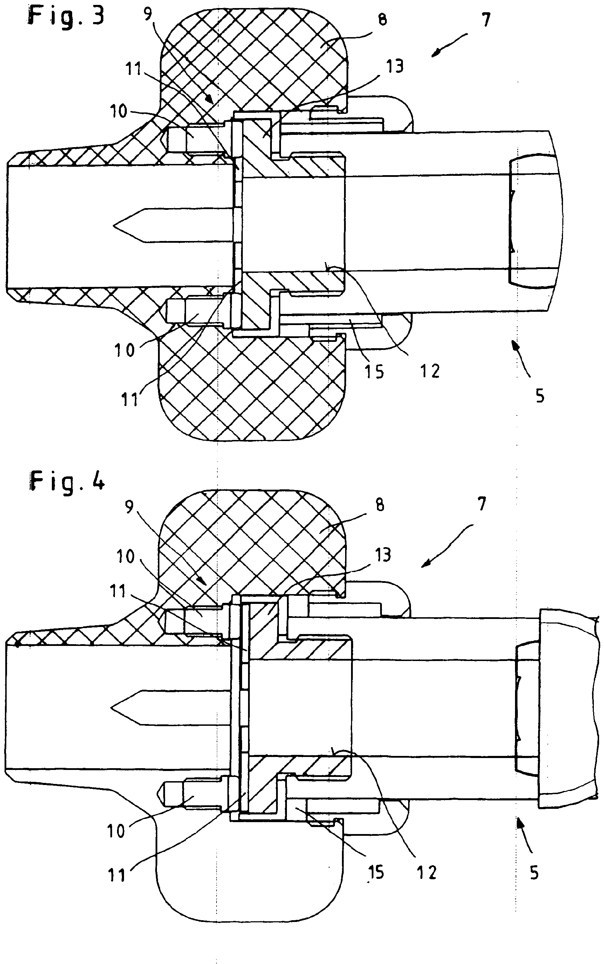

Der Aufbau des Antriebs 7 ist detaillierter insbesondere den Abbildungen Fig. 3 und 4 zu entnehmen.

Der Antrieb 7 dieses medizinischen Instruments 1 zeichnet sich durch einen besonderen Schutzmechanismus gegen das versehentliche Verdrehen des Stellrads 8 und somit des Schaftes 2 aus. Dieser Schutzmechanismus besteht darin, dass das Stellrad 8 zwischen einer die Verdrehung des Schaftes 2 um die Längsachse 6 des Schaftes 2 freigebenden Stellung und einer den Schaft 2 arretierenden Stellung in Längsrichtung des Schaftes 2 verlagerbar ist. Zu diesem Zweck ist der Antrieb 7 als sowohl um die Längsachse 6 des Schaftes 2 verdrehbares als auch in Längsrichtung des Schaftes 2 verlagerbares Stellrad 8 ausgebildet.

Nur in der in axialer Richtung des Schaftes 2 verlagerten Position des Stellrads 8 ist das Stellrad 8 auch mitsamt dem Schaft 2 um die Längsachse 6 des Schaftes 2 verdrehbar.

Das Arretieren des Schaftes 2 an der Handhabe erfolgt über am Stellrad 8 und an der Handhabe 5 ausgebildete, miteinander korrespondierende Rastelemente 9, über die der Schaft 2 in vorgegebenen Raststufen um die Längsachse 6 des Schaftes 2 verdrehbar an der Handhabe 5 festlegbar ist.

Bei der in Fig. 3 und 4 dargestellten Ausführungsform sind die Rastelemente 9 die als am Stellrad 8 angeordnete Raststifte 10 und an der Handhabe 5 ausgebildete Rastnuten 11 ausgebildet, wobei die Rastelemente 9 koaxial um die Längsachse 6 des Schaftes 2 angeordnet sind, da sich der hohle Schaft 2 in einer Durchgangsbohrung 12 in der Handhabe 5 fortsetzt, um weitere medizinische Instrumente vom proximalen Ende des medizinischen Instruments 1 her in den hohlen Schaft 2 einführen zu können.

Die Rastnuten 11 zur Aufnahme der Raststifte 10 sind bei der dargestellten Ausführungsform in einer am distalen Ende der Handhabe 5 angeordneten Rastscheibe 13 ausgebildet.

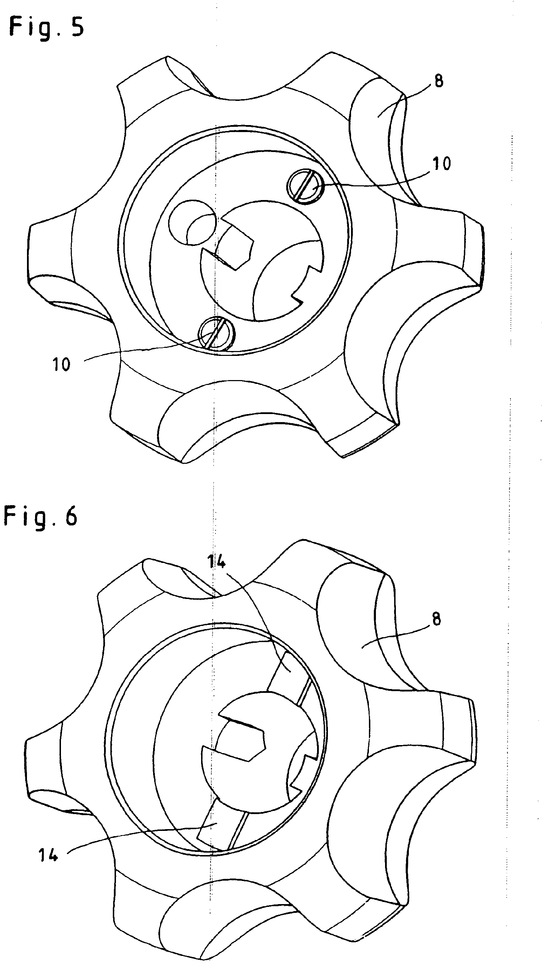

Die Abbildungen Fig. 5 und 6 zeigen zwei Ausführungsformen zur Ausbildung der mit den Rastnuten 11 korrespondierenden Rastelemente 9. Die in Fig. 5 dargestellte erste Ausführungsform entspricht der Darstellung gemäß den Abbildungen Fig. 3 und 4, bei denen die stellradseitigen Rastelemente 9 als Raststifte 10 ausgebildet sind. Bei der in Fig. 6 dargestellten zweiten Ausführungsform sind diese Rastelemente 9 als mit den Rastnuten 11 korrespondierende Rastnasen 14 ausgebildet.

Alternativ zu den dargestellten Ausführungsformen ist es selbstverständlich auch möglich, die Anordnung der Rastelemente 9 umzukehren, das heißt, die Raststifte 10 oder Rastnasen 14 an der Handhabe 5 und die Rastnuten 11 mitsamt der Rastscheibe 13 am Stellrad 8 auszubilden.

Um eine zusätzliche Sicherheit gegen ein versehentliches Verdrehen des Stellrads 8 und somit auch des Schaftes 2 zu schaffen, ist das Stellrad 8 gegen die Kraft eines Federelements 15 in Längsrichtung des Schaftes 2 verlagerbar. Das Stellrad 8 ist dabei über das Federelement 15 in Richtung auf die den Schaft 2 arretierende Stellung vorgespannt, so dass das Stellrad 8 automatisch in die arretierende Stellung zurückkehrt, wenn keine Kraft in axialer Richtung mehr auf das Stellrad 8 ausgeübt wird.

Der Antrieb 7 ist bei der zuvor beschriebenen Ausgestaltung unabhängig von einer anderweitigen Betätigung der Handhabe 5 in jeder Arbeitsposition im Einhandbetrieb betätigbar, da keine Wechselwirkung zwischen dem Antrieb 7 und der Betätigung der Griffteile 3 und 4 der Handhabe 5 besteht.

Die Handhabung des zuvor insbesondere anhand der Abbildungen Fig. 1, 3 und 4 beschriebenen medizinischen Instruments 1 geschieht wie folgt:

Fig. 3 zeigt den Antrieb 7 des medizinischen Instruments 1 in der den Schaft 2 an der Handhabe 5 arretierenden Stellung, in der die als Raststifte 10 ausgebildeten Rastelemente 9 des Stellrads 8 in die als Rastnuten 11 ausgebildeten Rastelemente 9 eingreifen, die in der Rastscheibe 13 angeordnet sind.

Um den Schaft 2 in eine neue Arbeitsposition zu verstellen, drückt der Bediener des medizinischen Instruments 1 das Stellrad 8 gegen die Kraft des Federelements 15 in axialer Richtung nach distal, bis die Raststifte 10 außer Eingriff mit den Rastnuten 11 treten, wie dies in Fig. 4 dargestellt ist. Ausschließlich in dieser axial verlagerten Verdrehstellung des Stellrads kann nunmehr das Stellrad 8 mitsamt dem Schaft 2 um die Längsachse 6 des Schaftes 2 verdreht werden, bis der Schaft 2 die gewünschte Position eingenommen hat.

Sobald der Bediener keinen Druck in axialer Richtung mehr auf das Stellrad 8 ausübt, zieht das Federelement 15 das Stellrad 8 wieder in axialer Richtung nach proximal zurück, bis die Raststifte 10 wieder in Eingriff mit den Rastnuten 11 treten und so den Schaft 2 wieder relativ zur Handhabe 5 arretieren, wie dies in Fig. 3 dargestellt ist.

Alternativ zur der dargestellten und zuvor beschriebenen Ausgestaltungsform ist es selbstverständlich auch möglich, die Axialverlagerung des Stellrads 8 umzukehren, das heißt, dass der Bediener das Stellrad 8 gegen die Kraft des Federelements 15 in axialer Richtung nach proximal zieht, um das Stellrad aus der arretierenden Stellung zu lösen. Sobald der Bediener keinen Zug in axialer Richtung mehr auf das Stellrad 8 ausübt, drückt das Federelement 15 das Stellrad 8 wieder in axialer Richtung nach distal zurück, bis die Raststifte 10 wieder in Eingriff mit den Ratnuten 11 treten und so den Schaft 2 wieder relativ zur Handhabe 5 arretieren.

Da das Betätigen des Antriebs 7 sowie das Lösen der durch die Rastelemente 9 und das Federelement 15 gebildeten Verdrehsicherung unabhängig von einer anderweitigen Betätigung der Handhabe 5 in jeder Arbeitsposition im Einhandbetrieb erfolgen kann, ist dieses medizinische Instrument 1 besonders vielseitig einsetzbar.

Ein wie zuvor beschrieben ausgebildetes medizinisches Instrument 1 zeichnet sich dadurch aus, dass es bei einfachem Aufbau und einfacher Handhabung eine größtmögliche Sicherheit gegen ein versehentliches Verdrehen des Schaftes 2 bietet.

### Bezugszeichenliste

- 1: medizinisches Instrument
- 2: Schaft
- 3: Griffteil
- 4: Griffteil
- 5: Handhabe
- 6: Längsachse
- 7: Antrieb
- 8: Stellrad
- 9: Rastelement
- 10: Raststift
- 11: Rastnut
- 12: Durchgangsbohrung
- 13: Rastscheibe
- 14: Rastnase
- 15: Federelement

## Patentansprüche

1. Medizinisches Instrument mit einem hohlen Schaft (2) und einer am proximalen Ende des Schaftes (2) angeordneten Handhabe (5), wobei der Schaft (2) über einen Antrieb (7) um seine Längsachse (6) verdrehbar ist und, wobei der Antrieb (7) als sowohl um die Längsachse (6) des Schaftes (2) verdrehbares als auch in Längsrichtung des Schaftes (2) verlagerbares Stellrad (8) ausgebildet ist,
**dadurch gekennzeichnet,**
**dass** der Antrieb (7) unabhängig von einer anderweitigen Betätigung der Handhabe (5) in jeder Arbeitsposition im Einhandbetrieb betätigbar ist.

2. Medizinisches Instrument nach Anspruch 1, **dadurch gekennzeichnet, dass** das Stellrad (8) zwischen einer die Verdrehung des Schaftes (2) um die Längsachse (6) des Schaftes (2) freigebenden Stellung und einer den Schaft (2) arretierenden Stellung in Längsrichtung des Schaftes (2) verlagerbar ist.

3. Medizinisches Instrument nach Anspruch 1 oder 2, **dadurch gekennzeichnet, dass** das Stellrad (8) gegen die Kraft mindestens eines Federelements (15) in Längsrichtung des Schaftes (2) verlagerbar ist.

4. Medizinisches Instrument nach Anspruch 3, **dadurch gekennzeichnet, dass** das Stellrad (8) über das mindestens eine Federelement (15) in Richtung auf die den Schaft (2) arretierende Stellung vorgespannt ist.

5. Medizinisches Instrument nach einem der Ansprüche 1 bis 4, **dadurch gekennzeichnet, dass** am Stellrad (8) und an der Handhabe (5) miteinander korrespondierende Rastelemente (9) ausgebildet sind, über die der Schaft (2) in vorgegebenen Raststufen um die Längsachse (6) des Schaftes (2) verdrehbar an der Handhabe (5) festlegbar ist.

6. Medizinisches Instrument nach Anspruch 5, **dadurch gekennzeichnet, dass** die Rastelemente (9) koaxial um die Längsachse (6) des Schaftes (2) angeordnet sind.

7. Medizinisches Instrument nach Anspruch 5 oder 6, **dadurch gekennzeichnet, dass** die Rastelemente (9) als am Stellrad (8) angeordnete Raststifte (10) oder Rastnasen (14) und an der Handhabe (5) ausgebildete Rastnuten (11) ausgebildet sind.

8. Medizinisches Instrument nach Anspruch 7, **dadurch gekennzeichnet, dass** die Rastnuten (11) in einer am distalen Ende der Handhabe (5) angeordneten Rastscheibe (13) ausgebildet sind.

9. Medizinisches Instrument nach einem der Ansprüche 1 bis 8, **dadurch gekennzeichnet, dass** der Schaft (2) zumindest abschnittweise gegenüber der Längsachse (6) des Schaftes (2) abgewinkelt ausgebildet ist.

10. Medizinisches Instrument nach einem der Ansprüche 1 bis 9, **dadurch gekennzeichnet, dass** der Antrieb (7) im Bereich des distalen Endes der Handhabe (5) angeordnet ist.

11. Medizinisches Instrument nach einem der Ansprüche 1 bis 9, **dadurch gekennzeichnet, dass** der Antrieb (7) im Bereich des proximalen Endes des Schaftes (2) angeordnet ist.
